# EUROPEAN PATENT APPLICATION

(11) **EP 4 155 731 A1**
(43) Date of publication of application: **29.03.2023**
(21) Application number: 21807995.2
(22) Date of filing: 23.04.2021
(51) Int. Cl.: G01N 33/15, G01N 33/50

(54) **EVALUATION METHOD FOR ALLERGEN INACTIVATOR AND EVALUATION KIT FOR ALLERGEN INACTIVATOR**

(30) Priority: 22.05.2020 JP 2020090066
(71) Applicant: School Corporation, Azabu Veterinary Medicine Educational Institution, Sagamihara-shi, Kanagawa 252-5201 (JP); ITEA Inc., Bunkyo-ku, Tokyo 113-0001 (JP)
(72) Inventor: SAKAGUCHI, Masahiro, Tokyo 113-0001 (JP); UCHIYAMA, Jumpei, Sagamihara-shi, Kanagawa 252-5201 (JP); YOSHIDA, Megumi, Tokyo 113-0001 (JP); MIZUKAMI, Keijiro, Sagamihara-shi, Kanagawa 252-5201 (JP); KURATA, Keigo, Tokyo 113-0001 (JP)
(74) Representative: Mewburn Ellis LLP
(86) International application number: PCT/JP2021/016529
(87) International publication number: WO 2021/235176

(57) **Abstract**

There are provided a method and a kit for evaluating allergen inactivators, the method and kit making it possible to carry out a precise and reproducible evaluation irrespective of the type of test drug, or the type of accompanying components thereof, that is subject to the evaluation.

A method for evaluating allergen inactivators, said method being characterized by comprising: a step for preparing an allergen-supporting membrane configured by supporting allergens on a membrane for supporting allergens; a step for treating the allergen-supporting membrane using a test drug; a step for washing the allergen-supporting membrane treated using the test drug, and subsequently treating the allergen-supporting membrane using antibodies specific to the allergens; a step for washing the allergen-supporting membrane treated using the antibodies specific to the allergens, and subsequently detecting the specific antibodies bound to the allergens; and a step for assessing the extent to which the allergens are inactivated by the test drug based on the detected quantity of the specific antibodies.

## Description

### TECHNICAL FIELD

The present invention relates to a method for evaluating allergen inactivators and a kit for evaluating allergen inactivators.

### BACKGROUND ART

In recent years, along with changes in home environments, such as a greater incidence of hermetic sealing and heat insulation or more widespread use of air-conditioning system, mites and house dust have become more prevalent, and the number of people troubled by allergy symptoms such as asthma attacks, atopic dermatitis, and rhinitis originating from this change has increased. Additionally, the number of people troubled by allergy symptoms originating from, *inter alia,* Japanese cedar pollen scattered through the air has also increased.

As one measure against allergic disease and other allergy symptoms, there has been an attempt to develop drugs for inactivating allergens serving as a source of these issues.

For example, Patent Document 1 discloses a method for removing allergens from an environment, the method being characterized in that the environment is treated using tannic acid. Patent Document 1 also indicates that when an extract from, *inter alia,* the grass *Phleum pratense,* the weed *Plantago lanceolata,* or house dust containing *Dermatophagoides pteronyssinus* is treated using a 1% aqueous solution of tannic acid, allergenicity can be mitigated in a cutaneous reaction test.

Additionally, Patent Document 2 discloses causing tannic acid, which is very safe for humans, to bind to fibers or a fiber product including fibers composed of cellulose via a cross-linking agent, thereby making it possible to prevent any reduction in the allergen inactivation performance of tannic acid even with repeated laundering.

It is also suggested that various substances other than the tannic acid described above are also effective. For example, Patent Document 3 discloses a use for a compound, or a salt thereof, that has surface activity and has a guanidino group as at least one cationic group per molecule. Additionally, Patent Document 4 discloses a use for an extract of pomegranate leaves produced using a solvent composed of at least one selected from water and hydrophilic solvents. In addition, Patent Document 5 discloses a use for an antiallergenic metal component such as silver or zinc. Additionally, Patent Document 6 discloses a use for a polymer compound having styrene sulfonic acid units.

### [Related Art Documents]

### [Patent Documents]

[Patent Document 1] Japanese Laid-Open Patent Application No. 61-44821
[Patent Document 2] Japanese Laid-Open Patent Application No. 2007-107149
[Patent Document 3] Japanese Laid-Open Patent Application No. 9-157152
[Patent Document 4] Japanese Laid-Open Patent Application No. 2002-370996
[Patent Document 5] Japanese Laid-Open Patent Application No. 2006-241431
[Patent Document 6] Japanese Laid-Open Patent Application No. 2009-155453

### DISCLOSURE OF THE INVENTION

### [Problems the Invention is Intended to Solve]

Typically, in development of allergen inactivators, when evaluating a drug expected to have inactivation performance, an attempt is made to prompt a reaction with allergens, and the quantity of allergens in a solution after the reaction is measured through, *inter alia,* a sandwich ELISA using allergen-specific antibodies. However, examples of drawbacks inherent in such a method include the issue that the ELISA for measuring the quantity of allergens is affected by the test drug, or accompanying components thereof, that is subject to the evaluation. Specifically, there have been cases in which, despite the allergens not being substantially inactivated, the quantity detected as the quantity of allergens measured through the ELISA is lower than the actual quantity due to the effect of the drug or the accompanying components thereof, resulting in the inactivation performance being over-evaluated. It is also thought that a component interfering with ELISA measurement could be removed using a procedure such as dialysis, but this approach is inefficient and not versatile for purposes such as screening large numbers of candidate substances.

It is accordingly an object of the present invention to provide a method and a kit for evaluating allergen inactivators, the method and kit making it possible to carry out a precise and reproducible evaluation irrespective of the type of test drug, or the type of accompanying components thereof, that is subject to the evaluation.

### [Means for Solving the Problems]

The inventors perfected the present invention upon having conducted numerous thoroughgoing investigations in order to achieve the aforementioned object.

Specifically, according to a first aspect, the present invention provides a method for evaluating allergen inactivators, said method being characterized by comprising: a step for preparing an allergen-supporting membrane configured by supporting allergens on a membrane for supporting allergens; a step for treating the allergen-supporting membrane using a test drug; a step for washing the allergen-supporting membrane treated using the test drug, and subsequently treating the allergen-supporting membrane using antibodies specific to the allergens; a step for washing the allergen-supporting membrane treated using the antibodies specific to the allergens, and subsequently detecting the specific antibodies bound to the allergens; and a step for assessing the extent to which the allergens are inactivated by the test drug based on the detected quantity of the specific antibodies.

In the method for evaluating allergen inactivators according to the first aspect of the present invention, it is possible to avoid the effects of the test drug or accompanying components thereof and to carry out a precise and reproducible evaluation because the test drug is used to treat the allergens supported on the allergen-supporting membrane, a washing treatment is carried out, and then detection is carried out using the antibodies specific to the allergens.

Additionally, according to a second aspect, the present invention provides a method for evaluating allergen inactivators, said method being characterized by comprising: a step for preparing, as a first allergen-supporting membrane, a membrane configured by supporting allergens on a first membrane for supporting allergens; a step for preparing a second allergen-supporting membrane configured by supporting allergens on a second membrane for supporting allergens in a quantity corresponding to the quantity of allergens supported on the first allergen-supporting membrane; a step for treating the first allergen-supporting membrane using a test drug; a step for washing the first allergen-supporting membrane treated using the test drug, and subsequently treating the first allergen-supporting membrane using antibodies specific to the allergens; a step for washing the first allergen-supporting membrane treated using the antibodies specific to the allergens, and subsequently detecting the specific antibodies bound to the allergens; a step for treating the second allergen-supporting membrane using a protein staining agent; a step for washing the second allergen-supporting membrane treated using the protein staining agent, and subsequently detecting the amount of staining by the protein staining agent, the amount corresponding to the quantity of allergens supported on the second allergen-supporting membrane; and a step for assessing the extent to which the allergens are inactivated by the test drug based on the relationship between the amount of staining and the detected quantity of the specific antibodies.

In the method for evaluating allergen inactivators according to the second aspect of the present invention, it is possible to avoid the effects of the test drug or accompanying components thereof and to carry out a precise and reproducible evaluation because the first allergen-supporting membrane, which is configured by supporting allergens on the first membrane for supporting allergens, is prepared, the test drug is used to treat the allergens supported on the first allergen-supporting membrane, a washing treatment is carried out, and then detection is carried out using the antibodies specific to the allergens. Additionally, it is possible to directly measure, by using the protein staining agent, a quantity corresponding to the quantity of allergens supported on the membrane supplied for detection using the specific antibodies because the second allergen-supporting membrane, which is configured by supporting the allergens on a second membrane for supporting allergens in a quantity corresponding to the quantity of allergens supported on the first allergen-supporting membrane, is prepared, and the allergens supported on the second allergen-supporting membrane are detected using the protein staining agent. Moreover, it is possible to carry out a more precise and reproducible evaluation with reference to the quantity of allergens on the membrane as directly measured using the protein staining agent because the evaluation is carried out based on the relationship between the amount of staining obtained through detection using the protein staining agent and the detected quantity of the specific antibodies.

In the method for evaluating allergen inactivators according to the present invention, it is preferable that the second allergen-supporting membrane according to the second aspect, which is supplied to the step for treatment using the protein staining agent, is supplied to the step for treatment using the protein staining agent after having been subjected to a step for treatment using the test drug. Accordingly, when the aforementioned method for evaluating allergen inactivators according to the second aspect of the present invention is implemented, it is possible to carry out an even more precise and reproducible evaluation with reference to the quantity of allergens supported on the membrane after treatment using the test drug because the allergens supported on the second allergen-supporting membrane are detected using the protein staining agent after having been treated using the test drug.

In the method for evaluating allergen inactivators according to the present invention, it is preferable that the protein staining agent according to the second aspect is a gold staining agent or a high-sensitivity CBB staining agent. Accordingly, when the aforementioned method for evaluating allergen inactivators according to the second aspect of the present invention is implemented, it is possible to detect, with higher sensitivity, the amount of staining corresponding to the quantity of allergens supported on the second allergen-supporting membrane.

In the method for evaluating allergen inactivators according to the present invention, it is preferable that the membrane for supporting allergens according to the first or second aspect is a PVDF-based membrane or a nitrocellulose-based membrane. Accordingly, it is possible to reliably support allergens on the membrane for supporting allergens and to detect, with excellent sensitivity and by using the specific antibodies or the protein staining agent, the allergens supported on the membrane for supporting allergens.

In the method for evaluating allergen inactivators according to the present invention, it is preferable that the allergens are supported, across a plurality of locations with a varied quantity of allergens, on a single membrane for supporting allergens according to the first or second aspect. Accordingly, it is possible to investigate allergen quantity conditions that are optimal for evaluation, or to investigate a plurality of allergen quantities at once, through operations on a single membrane, which is efficient.

In the method for evaluating allergen inactivators according to the present invention, it is preferable that a plurality of types of allergens are supported, across a plurality of locations, on a single membrane for supporting allergens according to the first or second aspect. Accordingly, it is possible to evaluate inactivation performance with respect to a plurality of types of allergens through operations on a single membrane, which is efficient. The plurality of types of allergens may be supported on the single membrane for supporting allergens across a plurality of locations with a varied quantity of allergens.

In the method for evaluating allergen inactivators according to the present invention, it is preferable that the membrane for supporting allergens according to the first or second aspect is formed in sections across a plurality of locations on a plate, and is caused to support the allergens across a plurality of locations formed in sections on the plate with a varied quantity of allergens. Accordingly, it is possible to investigate allergen quantity conditions that are optimal for evaluation, or to investigate a plurality of allergen quantities at once, through operations on a single plate, which is efficient.

In the method for evaluating allergen inactivators according to the present invention, it is preferable that the membrane for supporting allergens according to the first or second aspect is formed in sections across a plurality of locations on a plate, and is caused to support a plurality of types of allergens across a plurality of locations formed in sections on the plate. Accordingly, it is possible to evaluate inactivation performance with respect to a plurality of types of allergens through operations on a single plate, which is efficient. The plurality of types of allergens may be supported on the membrane for supporting allergens, which is formed in sections across a plurality of locations on the plate, across a plurality of locations with a varied quantity of allergens.

Additionally, according to a third aspect, the present invention provides a kit for evaluating allergen inactivators, said kit comprising a membrane for supporting allergens, specific antibodies for detecting allergens, and a protein staining agent.

The kit for evaluating allergen inactivators according to the present invention comprises the membrane for supporting allergens, the specific antibodies for detecting allergens, and the protein staining agent, thereby further simplifying the aforementioned implementation of the method for evaluating allergen inactivators according to the second aspect of the present invention.

In the kit for evaluating allergen inactivators according to the present invention, it is preferable that the protein staining agent is a gold staining agent or a high-sensitivity CBB staining agent. Accordingly, when the aforementioned method for evaluating allergen inactivators according to the second aspect of the present invention is implemented, it is possible to detect, with higher sensitivity, the amount of staining corresponding to the quantity of allergens supported on the second allergen-supporting membrane.

In the kit for evaluating allergen inactivators according to the present invention, it is preferable that the membrane for supporting allergens is a PVDF-based membrane or a nitrocellulose-based membrane. Accordingly, when the aforementioned method for evaluating allergen inactivators according to the second aspect of the present invention is implemented, it is possible to reliably support the allergens on the first or second membrane for supporting allergens, and to detect, with excellent sensitivity and by using the specific antibodies or the protein staining agent, the allergens supported on the membrane for supporting allergens.

In the kit for evaluating allergen inactivators according to the present invention, it is preferable that the membrane for supporting allergens is formed in the bottom surface of each of a plurality of wells in sections on a plate. Accordingly, because the membrane for supporting allergens is formed in sections across a plurality of locations on the plate, when the aforementioned method for evaluating allergen inactivators according to the second aspect of the present invention is implemented, it is possible to evaluate inactivation performance with respect to a plurality of types of allergens, to investigate allergen quantity conditions that are optimal for evaluation, or to investigate a plurality of allergen quantities at once, through operations on a single plate, which is efficient.

In the kit for evaluating allergen inactivators according to the present invention, it is preferable that allergens are supported in advance on the membrane for supporting allergens. Accordingly, because the allergen-supporting membrane is prepared, when the aforementioned method for evaluating allergen inactivators according to the second aspect of the present invention is implemented, the method can be more efficiently implemented.

### [Effect of the Invention]

According to the present invention, it is possible to provide a method and a kit for evaluating allergen inactivators, the method and kit making it possible to carry out a precise and reproducible evaluation irrespective of the type of test drug, or the type of accompanying components thereof, that is subject to the evaluation.

### BRIEF DESCRIPTION OF THE DRAWINGS

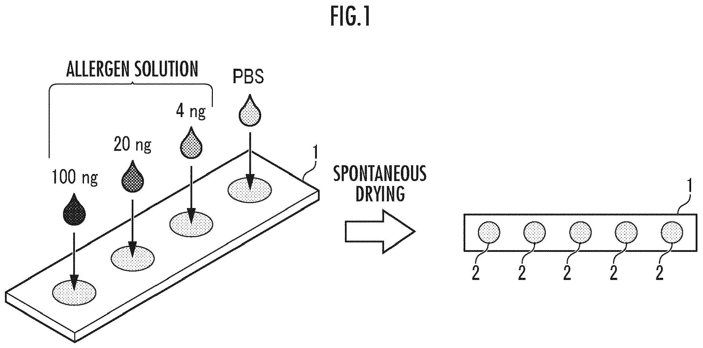

FIG. 1 is a schematic diagram showing one embodiment of an allergen-supporting membrane used in the present invention;

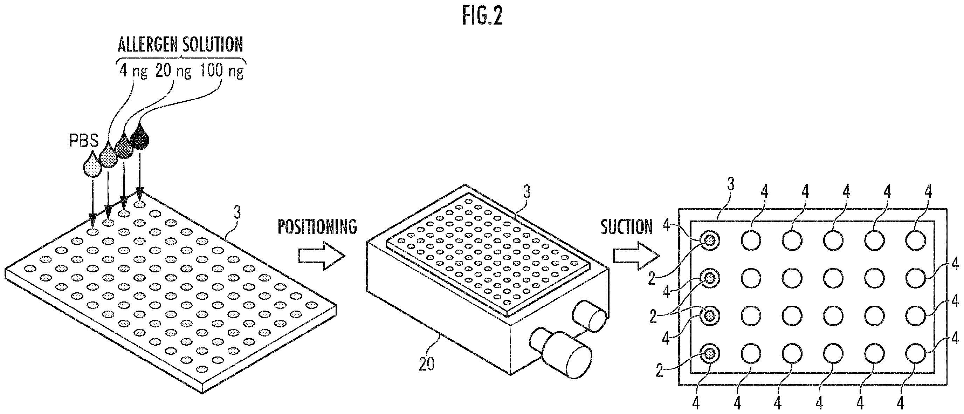

FIG. 2 is a schematic diagram showing another embodiment of the allergen-supporting membrane used in the present invention;

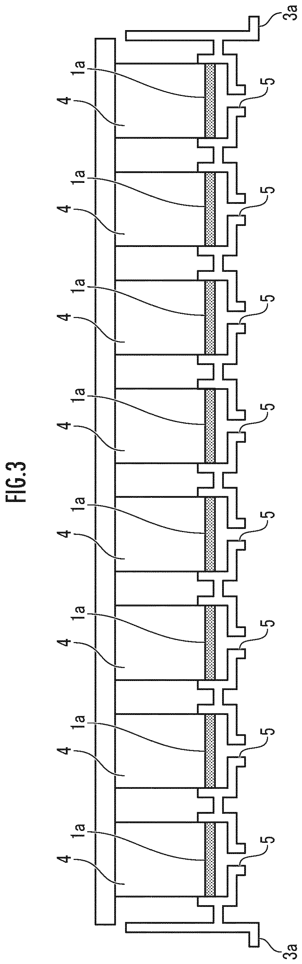

FIG. 3 is a schematic diagram showing the cross-sectional shape of a multiscreen plate 3 shown in FIG. 2;

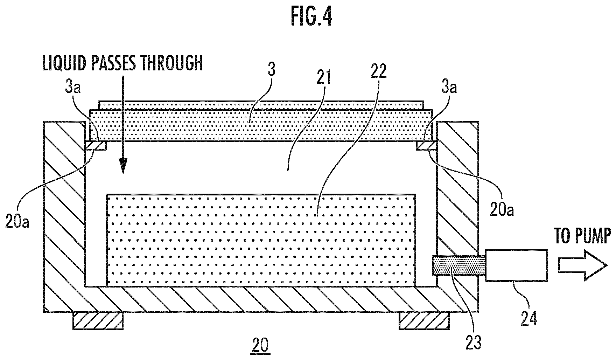

FIG. 4 is a schematic diagram showing the cross-sectional shape of a vacuum manifold 20 shown in FIG. 2;

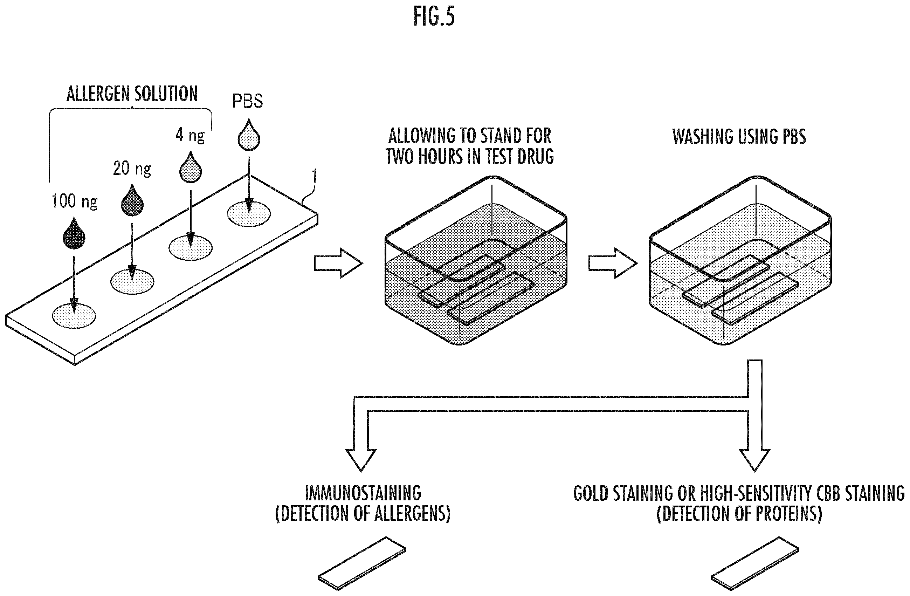

FIG. 5 is a schematic diagram illustrating a procedure for operations in test examples 1 to 7;

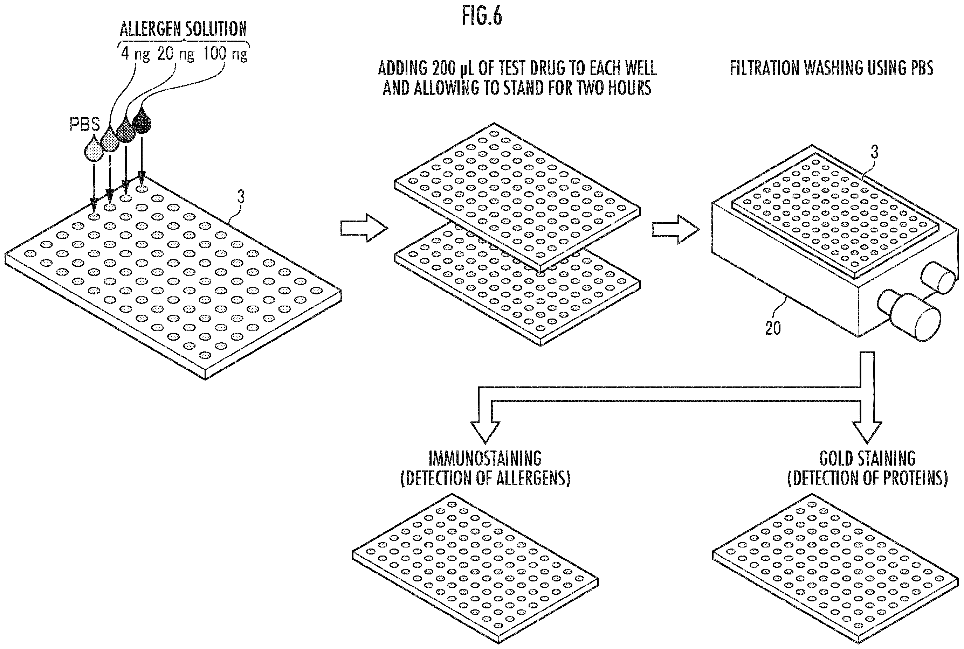

FIG. 6 is a schematic diagram illustrating a procedure for operations in test examples 8 and 9;

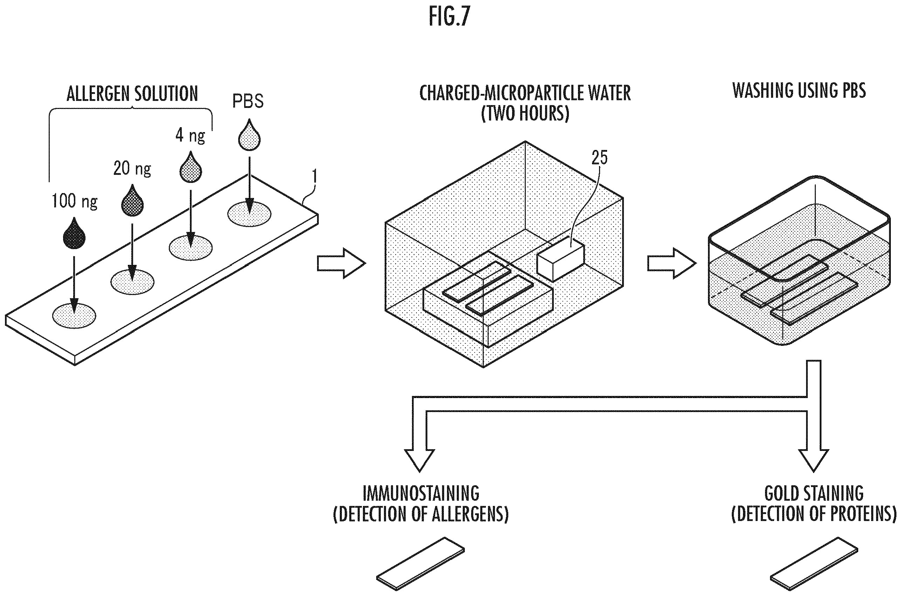

FIG. 7 is a schematic diagram illustrating a procedure for operations in test example 10;

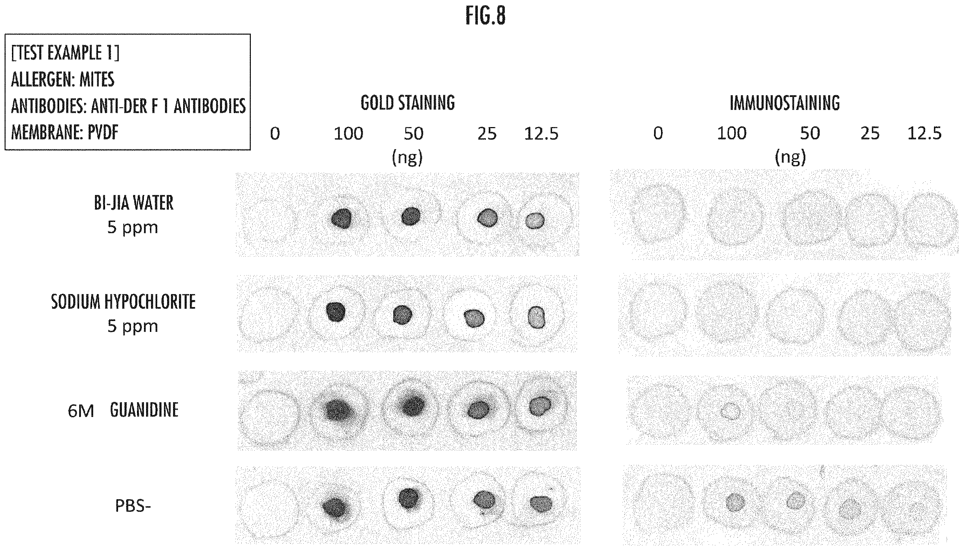

FIG. 8 is a chart showing results obtained in test example 1 by supporting purified mite allergens on a PVDF membrane, treating the allergens using respective drugs being examined, and subsequently carrying out protein staining using a gold stain or immunostaining using anti-Der f1 monoclonal antibodies;

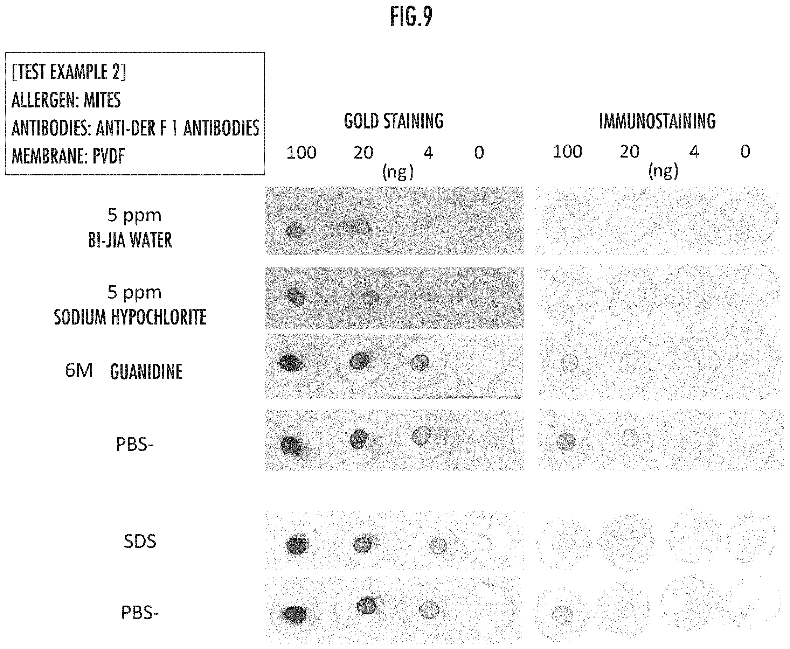

FIG. 9 is a chart showing results obtained in test example 2 by supporting purified mite allergens on a PVDF membrane, treating the allergens using respective drugs being examined, and subsequently carrying out protein staining using a gold stain or immunostaining using anti-Der f1 monoclonal antibodies;

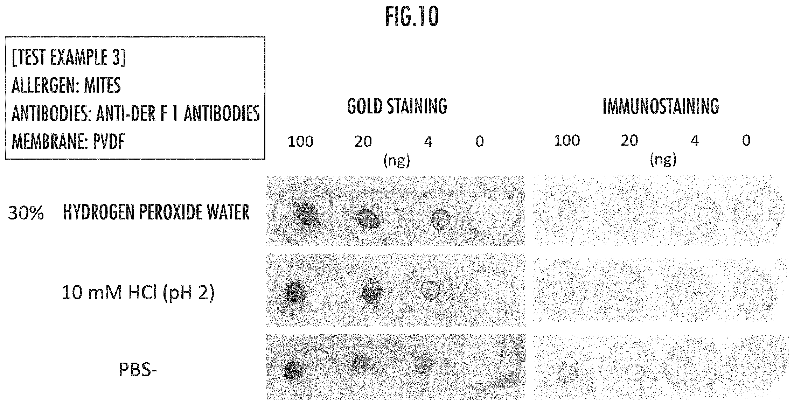

FIG. 10 is a chart showing results obtained in test example 3 by supporting purified mite allergens on a PVDF membrane, treating the allergens using respective drugs being examined, and subsequently carrying out protein staining using a gold stain or immunostaining using anti-Der f1 monoclonal antibodies;

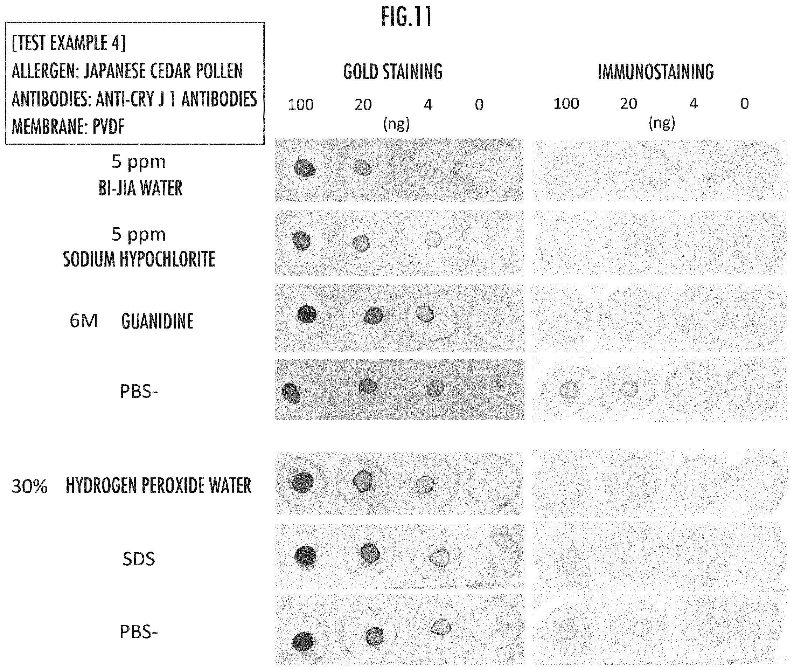

FIG. 11 is a chart showing results obtained in test example 4 by supporting purified Japanese cedar pollen allergens on a PVDF membrane, treating the allergens using respective drugs being examined, and subsequently carrying out protein staining using a gold stain or immunostaining using anti-Cry j1 monoclonal antibodies;

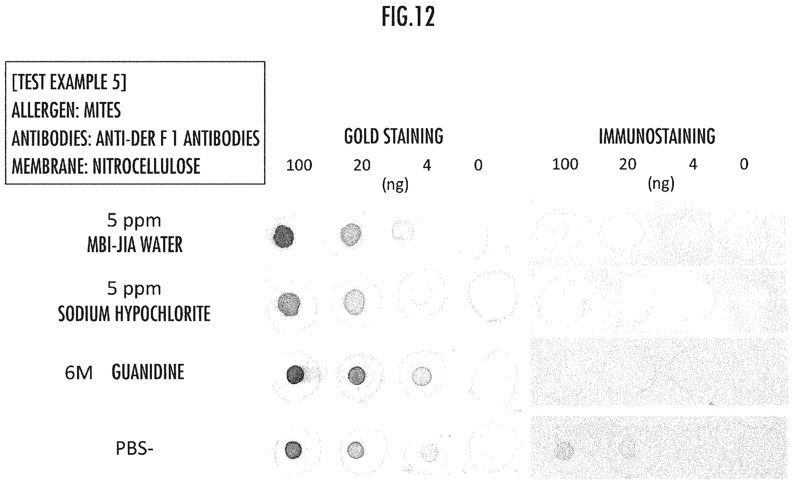

FIG. 12 is a chart showing results obtained in test example 5 by supporting purified mite allergens on a nitrocellulose membrane, treating the allergens using respective drugs being examined, and subsequently carrying out protein staining using a gold stain or immunostaining using anti-Der f1 monoclonal antibodies;

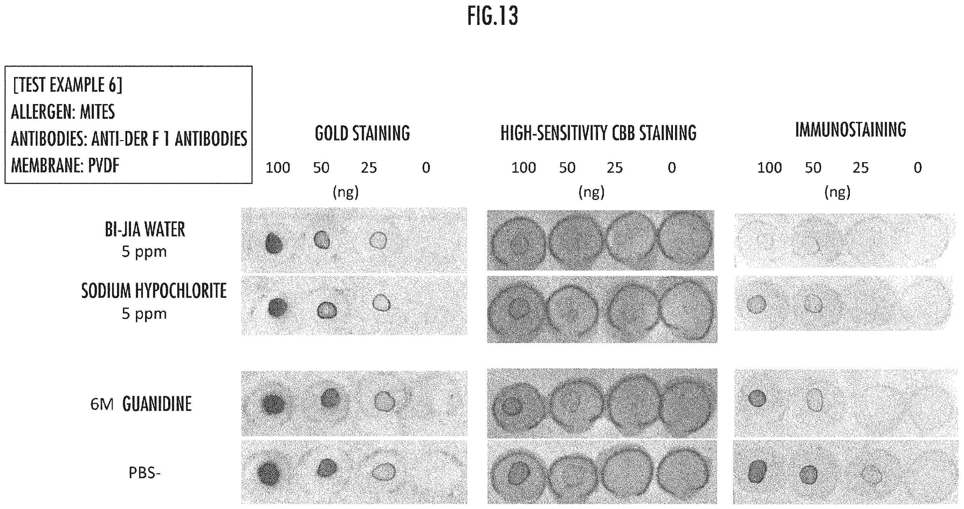

FIG. 13 is a chart showing results obtained in test example 6 by supporting purified Japanese cedar pollen allergens on a PVDF membrane, treating the allergens using respective drugs being examined, and subsequently carrying out protein staining using a gold stain or a high-sensitivity CBB stain, or immunostaining using anti-Cry j1 monoclonal antibodies;

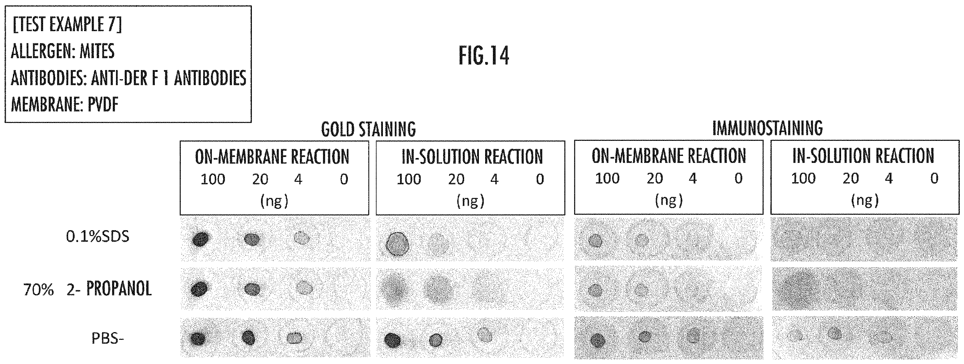

FIG. 14 is a chart showing (1) results obtained in test example 7 by supporting purified mite allergens on a PVDF membrane, treating the allergens using respective drugs being examined, and subsequently carrying out protein staining using a gold stain or immunostaining using anti-Der f1 monoclonal antibodies, and (2) results obtained in test example 7 by mixing purified mite allergens and respective drugs being examined within a liquid, subsequently blotting the mixtures onto a PVDF membrane, and carrying out protein staining using a gold stain or immunostaining using anti-Der f1 monoclonal antibodies;

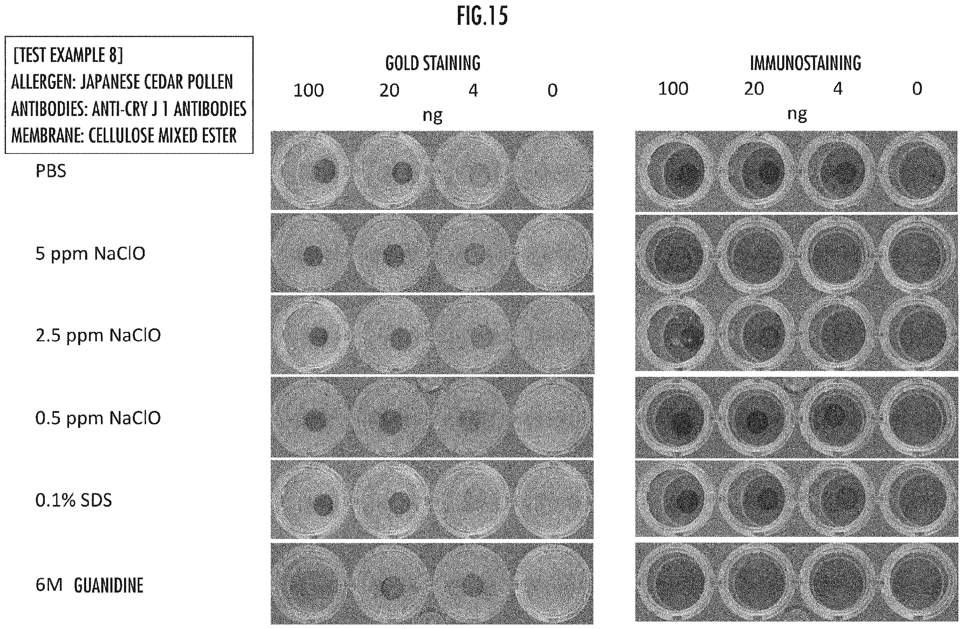

FIG. 15 is a chart showing results obtained in test example 8 by supporting purified Japanese cedar pollen allergens on filter membranes composed of a mixed cellulose ester (substance resembling nitrocellulose) in respective wells of a multiscreen filter plate, treating the allergens using respective drugs being examined, and subsequently carrying out protein staining using a gold stain or immunostaining using anti-Cry j1 monoclonal antibodies;

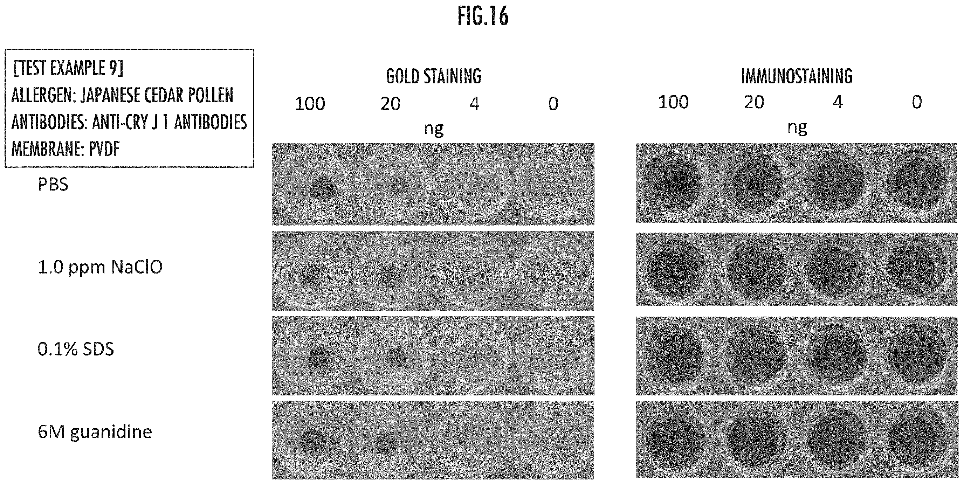

FIG. 16 is a chart showing results obtained in test example 9 by supporting purified Japanese cedar pollen allergens on filter membranes composed of PVDF in respective wells of a multiscreen filter plate, treating the allergens using respective drugs being examined, and subsequently carrying out protein staining using a gold stain or immunostaining using anti-Cry j1 monoclonal antibodies; and

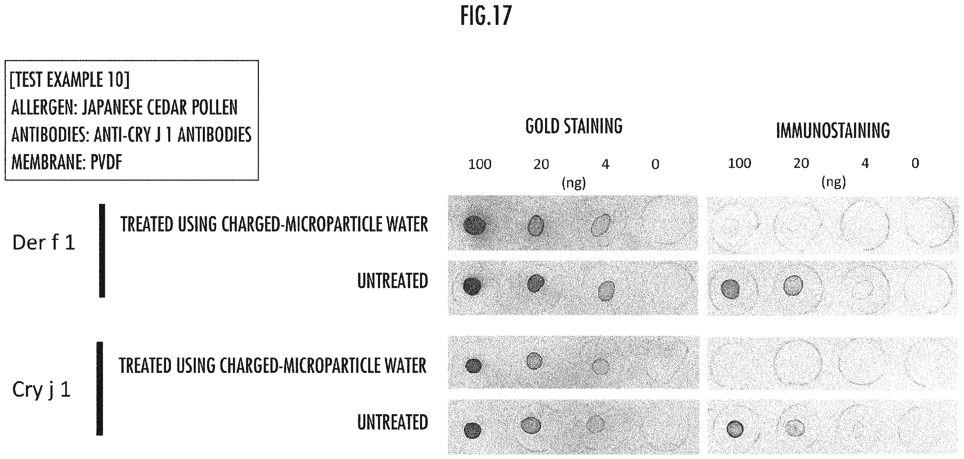

FIG. 17 is a chart showing results obtained in test example 10 by supporting purified mite allergens or purified Japanese cedar pollen allergens on a PVDF membrane, treating the allergens using charged-nanoparticle water containing OH radicals produced by applying a high voltage to water in air, and subsequently carrying out protein staining using a gold stain, immunostaining using anti-Der f1 monoclonal antibodies, or immunostaining using anti-Cry j1 monoclonal antibodies.

### MODE FOR CARRYING OUT THE INVENTION

The present invention provides an evaluation method and an evaluation kit for measuring the extent to which a test drug has allergen-inactivating performance. The "test drug" may be any substance that an evaluator desires to evaluate. "Allergens" are substances that induce allergies in humans or animals; in the present invention, any allergens selected by an evaluator from among allergens may be employed.

It is preferable to use, as a membrane for supporting allergens used in the present invention, a membrane that is capable of supporting allergens as a solid phase and enabling detection of the allergens on the solid phase. The mode of support used may involve directly supporting allergens on the membrane using characteristics unique to the membrane, or a mode involving supporting allergens on the membrane with a prescribed cross-linking structure interposed therebetween may be employed. A particularly preferred example of the membrane includes membrane-filter-type membranes. For example, it is possible to use a membrane having a pore diameter of about 0.2-1.2 µm, a thickness of about 80-200 µm, and a void ratio of about 60-90%. Specific examples include PVDF-based membranes composed of a material including polyvinylidene difluoride (PVDF), and nitrocellulose membranes composed of a material including nitrocellulose, although the membrane is not particularly limited to these examples. These membranes make it possible to efficiently support allergens which are configured from peptides or proteins directly on the membrane.

The quantity of allergens supported on the membrane is to be set, as appropriate, in accordance with the sensitivity of detection by specific antibodies that shall be described later or with the sensitivity of detection through protein staining. Typically, when an allergen-containing solution is poured to support in a dot formation within a range of about 0.04-0.09 cm², the quantity of allergens supported is preferably 2 ng or more and 500 ng or less, and more preferably 4 ng or more and 100 ng or less.

In the present invention, an allergen-supporting membrane on which the aforementioned allergens are supported is treated using a test drug, after which the supported allergens on the treated membrane are detected using antibodies specific to the allergens. In this procedure, when the properties of the allergens enabling recognition by the specific antibodies weakens due to, *inter alia,* a change in the structure of the allergens, an allergen-antibody reaction that induces allergy symptoms also weakens. Thus, the extent to which allergens are inactivated can be assessed based on the detected quantity of specific antibodies that bind to the allergens and are detected on the membrane after treatment using the test drug. For example, if the detected quantity is about the same as that for a control that is not treated using the test drug, it can be assessed that inactivation does not occur due to the test drug. However, if the specific antibodies cannot be detected, it can be assessed that inactivation occurs due to the test drug. Additionally, when, e.g., the detected quantity is 30-70% of that for a control that is not treated using the test drug, it can be assessed that inactivation occurs to an intermediate extent relative to when the specific antibodies cannot be detected.

Conditions pertaining to drug concentration, solvent, treatment temperature, treatment time, etc., in the treatment using the test drug are to be set in a discretionary manner by an evaluator. For example, as the mode of treatment, it is possible to carry out treatment by, *inter alia*: immersing the allergen-supporting membrane, for a fixed time, in a solution in which a drug being tested is dissolved or dispersed; spraying the allergen-supporting membrane with the aforementioned solution and allowing the allergen-supporting membrane to stand for a fixed time; or dispersing the drug being tested in a prescribed space by forming the drug being tested into microparticles or a gas and subsequently allowing the allergen-supporting membrane to stand for a fixed time inside the space. There is no particular limitation as to the form of the substance being tested. The substance being tested may be a composition such as a solution containing a substance selected in a discretionary manner by an evaluator, or a sprayed, gaseous, microparticulate, or ionized form of the solution. Alternatively, it is permissible to apply, e.g., nano-sized charged-microparticle water containing OH radicals produced by applying a high voltage to water in air, stable ionized forms of oxygen/water molecules generated through plasma discharge, microparticles of an aqueous solution of hypochlorous acid produced by electrolyzing saline, or another substance having an effect in sterilization of spaces, virus removal, decontamination, deodorization, etc., when evaluating the allergen-inactivating performance of the substance.

After the treatment using the test drug, washing is carried out using phosphate-buffered saline (PBS), etc., in order to separate the test drug or accompanying components thereof from the membrane. There is no particular limitation as to the mode of washing, provided that the test drug or accompanying components thereof can be separated from the membrane. One example of such a mode is a method in which, *inter alia*: the allergen-supporting membrane after treatment using the drug being tested is placed in a container into which a washing solution such as PBS has already been introduced; the allergen-supporting membrane is immersed in the washing solution, and then is either allowed to stand or shaken for a fixed time; and the washing solution is then removed. Alternatively, in a multiscreen filter plate used in examples that shall be described later, because the membrane is formed on the bottom-surface sides of a plurality of wells in sections on the plate, it is possible to implement a washing treatment by amassing the washing solution in the wells, subsequently positioning the plate in a vacuum manifold provided with a plate for recovering washing solution from each plate, and suctioning the washing solution. A single cycle of washing may be performed, but it is preferable to repeat the operation two or three times for the purpose of effective washing.

There is no particular limitation as to the specific antibodies used in the present invention, provided that the specific antibodies specifically recognize allergens. The feature of "specifically recognizing allergens" involves being provided with properties for selectively binding to specific allergens; this definition would naturally be understood by an ordinary person skilled in the art. For example, the specific antibodies can be in the form of antiserum, an IgG fraction, polyclonal antibodies, monoclonal antibodies, or another form. It is possible to use, as a method for formulating the specific antibodies, any well-known means in the relevant technical field. For example, it is possible to formulate antiserum, an IgG fraction, polyclonal antibodies, etc., by administering allergens to an immunized animal and harvesting blood from the animal. Additionally, it is possible to formulate monoclonal antibodies by isolating cells that produce specific antibodies through hybridoma formulation. Because specific antibodies against mite allergens and Japanese cedar pollen allergens are commercially available, such commercial products can be used, as in the examples that shall be described later.

In a discretionary non-limiting aspect of the present invention, two sets of allergen-supporting membranes configured to support the allergens may be prepared, with one membrane being supplied for detection using the specific antibodies and the other membrane being supplied for detection using a protein staining agent. Accordingly, it is possible to more precisely assess the extent to which the allergens are inactivated by the test drug with reference to the quantity of supported allergens because a quantity corresponding to the quantity of allergens supported on the membrane supplied for detection using the specific antibodies is detected by using the protein staining agent while in the same state of being supported on a membrane. For example, even when the type or batch of allergens changes despite the allergens being common, or when the type or batch of membranes being used changes, or when the type or batch of specific antibodies being used changes, it is possible to objectively ascertain the situation with reference to the quantity of allergens supported on the membrane, and it is also possible to, e.g., correct a quantity detected using the specific antibodies by using the amount of staining by the protein staining agent, as necessary.

In a discretionary non-limiting aspect of the present invention, two sets of allergen-supporting membranes configured to support the allergens may be prepared, with one membrane being supplied for detection using the specific antibodies and the other membrane being supplied for detection using a protein staining agent, in the same manner as with the membrane supplied for detection using the specific antibodies, after having been treated using a test drug. Accordingly, it is possible to even more precisely assess the extent to which the allergens are inactivated by the test drug with reference to the quantity of allergens supported on the membrane after treatment using the test drug because a quantity corresponding to the quantity of allergens supported on the membrane supplied for detection using the specific antibodies is detected by using the protein staining agent while in the same state of being supported on a membrane after having been treated using the test drug. For example, when the temporarily supported allergens peel away from the membrane due to the treatment using the test drug, it is possible to ascertain a state with respect to the quantity of allergens, etc., and it is also possible to, e.g., correct the quantity detected using the specific antibodies by using the amount of staining by the protein staining agent, as necessary.

In the detection using the specific antibodies, it is possible to use methods well known to persons skilled in the art. One example includes immersing the allergen-supporting membrane in a specific-antibody-containing solution, allowing the allergen-supporting membrane to stand or shaking the same for a fixed time before washing the allergen-supporting membrane using a washing solution such as PBS and separating specific antibodies that are not bound to the allergens on the membrane, subsequently treating in the same manner a solution that contains labeled secondary antibodies to the specific antibodies, and then chromatically or optically detecting an image (detected quantity of specific antibodies) obtained using the marker of the secondary antibodies. The washing after the treatment using the specific antibodies or the labeled secondary antibodies can be carried out in the same manner as the washing after the treatment of the allergen-supporting membrane using the test drug described above. Examples of the marker include horse radish peroxidase (HRP), β-galactosidase, fluorescence, and radioisotopes.

In the detection using the protein staining agent, it is possible to use methods well known to persons skilled in the art. One example includes immersing the allergen-supporting membrane in a protein-staining-agent-containing solution, allowing the allergen-supporting membrane to stand or shaking the same for a fixed time before washing the allergen-supporting membrane using a washing solution such as PBS and separating protein staining agent that is not bound to the allergens on the membrane, and then chromatically or optically detecting an image (amount of staining by protein staining agent) obtained using the protein staining agent. The washing after the treatment using the protein staining agent can be carried out in the same manner as the washing after the treatment of the allergen-supporting membrane using the test drug described above. Examples of the protein staining agent include gold stain, high-sensitivity CBB stains, silver stain, India ink, reactive dyes, and fluorescent pigment stains.

FIG. 1 shows one embodiment for preparing the allergen-supporting membrane configured by supporting the allergens on the membrane for supporting allergens in the present invention. In the embodiment shown in FIG. 1, a prescribed amount of an allergen-containing PBS solution prepared by changing concentrations is added dropwise to a membrane 1 cut into a rectangular strip, after which the membrane 1 is allowed to spontaneously dry to prepare an allergen-supporting membrane in which a plurality of allergens 2 are supported in a dot formation in amounts of 100 ng, 20 ng, and 4 ng. Additionally, PBS is added dropwise to a non-allergen-containing control. In this procedure, the allergens may be supported at the locations of the plurality of dots in varied quantities, or the allergens may be supported at these locations in varied types.

FIG. 2 shows another embodiment for preparing the allergen-supporting membrane configured by supporting the allergens on the membrane for supporting allergens in the present invention. In the embodiment shown in FIG. 2, a multiscreen filter plate 3 is used. A vacuum manifold 20 in which the multiscreen filter plate 3 is positioned is also used. FIGS. 3 and 4 show the cross-sectional shapes of the multiscreen plate 3 and the vacuum manifold 20, respectively. A well hole 5 is present on the bottom-surface side of each well 4 in the multiscreen filter plate 3, and a membrane 1a for supporting the allergens is formed so as to block each of the well holes 5. The vacuum manifold 20 is provided with a flexible flange part 20a such that an end section 3a of the multiscreen filter plate 3 can be placed on the vacuum manifold 20 in a close state that does not allow passage of air. Furthermore, a space 21 is provided on the lower side partitioning the multiscreen filter plate 3 thus placed, the space 21 being linked to a suction pump (not shown) via a connector 24 linked to a suction port tube 23. A configuration is adopted in which, when suction produced by the suction pump creates negative pressure inside the space 21, a solution introduced into each of the wells 4 in the multiscreen filter plate 3 can be suctioned and removed to the lower side of the well holes 5 through the water-permeable membranes 1a. Additionally, a waste liquid recovery container 22 is disposed inside the space 21, and the solution suctioned and removed from the multiscreen filter plate 3 is recovered as a waste liquid in the waste liquid recovery container 22.

In the embodiment shown in FIG. 2, a prescribed amount of an allergen-containing PBS solution prepared by changing concentrations is added dropwise to a membrane 1a formed on the bottom-surface sides of each of the wells 4 in the multiscreen filter plate 3, after which the membrane 1a is allowed to spontaneously dry or is positioned in the vacuum manifold 20 and suctioned through filtration to prepare an allergen-supporting membrane in which a plurality of allergens 2 are supported in a dot formation in amounts of 100 ng, 20 ng, and 4 ng. Additionally, PBS is added dropwise to a non-allergen-containing control. In this procedure, the allergens may be supported on the plurality of membranes 1a in each of the plurality of wells 4 in varied quantities, or the allergens may be supported on the plurality of membranes 1a in varied types. In the multiscreen filter plate 3, because the membranes are formed in sections across a plurality of locations on a single plate by well walls 4a, it is possible to treat each of the membranes 1a corresponding to each of the regions formed in sections using respectively different types of solutions. It is also possible to determine conditions or carry out screening of a plurality of allergens or inactivators to be evaluated using a single operation. Additionally, it is possible to wash the allergen-supporting membranes by positioning the plate 3 in the vacuum manifold 20, amassing the washing solution such as PBS within each of the plurality of wells 4, and suctioning the washing solution. Furthermore, with respect to not only a treatment for supporting allergens but also a treatment carried out using the test drug, a treatment carried out using specific antibodies or secondary antibodies, or a washing treatment carried out after the aforementioned treatments, it is possible to carry out the treatments on the membranes 1a on the bottom-surface sides within each of the wells 4 formed in sections across a plurality of locations on the multiscreen filter plate 3, in the same manner as with the treatment for supporting the allergens described above on the membranes 1a, using the prescribed solutions for these treatments.

From another standpoint, the present invention provides an evaluation kit that is suitably used in implementing the method for evaluating allergen inactivators described above. Specifically, this evaluation kit is provided with the membrane for supporting allergens, the specific antibodies for detecting allergens, and the protein staining agent, from among the elements used in the evaluation method described above. However, the evaluation kit may also include other elements from among the elements used in the evaluation method described above, with the aforementioned elements constituting a minimum configuration. Examples of other elements include the washing solution for washing the membrane and the labeled secondary antibodies for detecting the specific antibodies. The preferred aspects of these elements are as described above. The evaluation kit may also include elements not set forth in the present description.

In the evaluation kit, it is preferable that allergens are supported in advance on the membrane for supporting allergens. Accordingly, it is possible to more efficiently implement the method for evaluating allergen inactivators because the allergen-supporting membrane is prepared.

In the evaluation kit, it is preferable that the membrane for supporting allergens is formed on the bottom surface of each of a plurality of wells in sections on a plate. Accordingly, because membranes for supporting allergens are formed in sections across a plurality of locations on the plate, it is possible to more efficiently implement the method for evaluating allergen inactivators. The evaluation kitcan also be applied to mass-processable automation. Such a configuration can be implemented using, e.g., the multiscreen filter plate described above.

Any allergens may be applied in the present invention, as mentioned above; however, conventional examples include allergens derived from mites, pollen, foods, cockroaches, mold, animals, etc. For example, important mites serving as factors in mite allergies include those of the genus *Dermatophagoides* belonging to the family *Pyroglyphidae,* more specifically *Dermatophagoides farinae, Dermatophagoides pteronyssinus,* etc. Der f1, Der p1, etc., are known as main allergens separated from *Dermatophagoides farinae* and *Dermatophagoides pteronyssinus.* Additionally, Cry j1, Cryj 2, etc., are known as main allergens separated from Japanese cedar pollen.

### [Examples]

Examples are given below to provide a more specific description of the present invention. However, these examples in no way limit the scope of the present invention.
(1. Test drug)
   (1-a) 5 ppm Bi-jia water (slightly acidic sodium hypochlorite, pH 5.5)
   (1-b) 5 ppm sodium hypochlorite (pH 11-12)
   (1-c) 6M guanidine
   (1-d) 0.1 w/v% SDS
   (1-e) 30 v/v% hydrogen peroxide water
   (1-f) 10 mM hydrochloric acid (pH 2)
   (1-g) 70 v/v% 2-propanol-containing water
(2. Allergens and antibodies specific thereto)
   (2-1. Mites)
   (2-a) Purified mite allergens: product name "Der f1" (made by ITEA Inc.) (origin: *Dermatophagoides farinae)*
   (2-b) Anti-Der f1 monoclonal antibodies: 5F2.1.8 (made by ITEA Inc.)
   (2-2. Japanese cedar)
   (2-c) Purified Japanese cedar pollen allergens: product name "Cry j1" (made by ITEA Inc.) (origin: Japanese cedar (Cryptomeria japonica))
   (2-d) Anti-Cry j1 monoclonal antibodies: KW-S131 (made by ITEA Inc.)
(3. Formulation of allergen-supporting membrane)
   (3-1. PVDF membrane)
      - A PVDF membrane (pore diameter: 0.45 µm) was immersed in methanol to be made hydrophilic, after which a prescribed quantity of allergens was blotted onto the PVDF membrane in a dot formation (0-100 ng/dot).
   (3-2. Nitrocellulose membrane)
      - A prescribed quantity of allergens was blotted onto a nitrocellulose membrane (pore diameter: 0.45 µm) in a dot formation (0-100 ng/dot).
(4. Treatment using test drug)
   - The allergen-supporting membrane was washed three times using PBS (-).
   - The membrane was placed in a plastic container and immersed in 5 mL of a solution containing the test drug.
   - The membrane was allowed to stand for two hours at normal temperature.
   - The solution of the test drug was discarded, and the membrane was washed three times using PBS (-).
   - Protein staining or immunostaining was carried out.
(5. Protein staining)
   (5-1. Gold staining)
      A gold stain kit (product name "Gold protein detection kit for membranes," made by Cytodiagnostics Inc.) was used.
      - The allergen-supporting membrane (after "4. Treatment using test drug" described above) was immersed in a "1X wash buffer" included in the kit and was shaken for 15 minutes.
      - The "1X wash buffer" was discarded, and the membrane was washed three times using distilled water.
      - The membrane was immersed in 2 mL of a "Gold Solution" included in the kit and was shaken for one to two hours at normal temperature.
      - The "Gold Solution" was discarded, and the membrane was washed twice using the "1X wash buffer" included in the kit.
      - The membrane was washed using a large amount of distilled water.
      - The membrane was allowed to spontaneously dry.
   (5-2. High-sensitivity CBB staining)
      A high-sensitivity CBB stain kit (made by Apro Life Science Institute, Inc.) was used.
      - The allergen-supporting membrane (after "4. Treatment using test drug" described above) was washed three times using PBS (-).
      - The membrane was allowed to spontaneously dry.
      - The membrane was immersed in 1 mL of a high-sensitivity CBB stain solution (made by Apro Life Science Institute, Inc.) and was shaken for five minutes at normal temperature.
      - The stain solution was discarded, and the membrane was decolorized for 30 minutes using an aqueous solution containing 10% methanol/10% acetic acid while the solution was frequently replaced.
      - The membrane was washed using distilled water and was allowed to spontaneously dry.
(6. Immunostaining)
   - The allergen-supporting membrane (after "4. Treatment using test drug" described above) was immersed in 1 w/v% gelatin-containing PBS and was blocked for ten minutes at 37°C.
   - The blocking liquid was discarded, and the membrane was washed three times using PBS (-).
   - Primary antibodies were diluted using 1 w/v% BSA-containing PBST (0.02 v/v% Tween-20) so as to reach a concentration of 1 pg/mL, 2 mL of the primary antibodies was added to the blocked membrane, and a reaction was carried out for 30 minutes at normal temperature while the membrane was shaken.

   - A primary antibody reaction solution was discarded, and the membrane was washed three times using PBST.
   - HRP-labeled secondary antibodies (product name "HRP-Goat anti-Mouse IgG," made by Zymed Laboratories) were diluted using 1 w/v% BSA-containing PBST (0.02 v/v% Tween-20) so as to reach a concentration of 3 pg/mL, 2 mL of the secondary antibodies was added to the membrane reacted with the primary antibodies, and a reaction was carried out for 30 minutes at normal temperature while the membrane was shaken.
   - A secondary antibody reaction solution was discarded, and the membrane was washed four times using PBST.
   - An amount of 1 mL of a coloring reagent solution (product name "Ez West Blue," made by ATTO Co., Ltd.) was added to the membrane reacted with the secondary antibodies, and a reaction was carried out for ten minutes.
   - The membrane was washed using a large amount of distilled water, and the reaction was stopped.
   - The membrane was allowed to spontaneously dry.

### <Test example 1>

The (1-a), (1-b), or (1-c) indicated below was used as the (1. Test drug) described above.
(1-a) 5 ppm of Bi-jia water (slightly acidic sodium hypochlorite, pH 5.5)
(1-b) 5 ppm of sodium hypochlorite (pH 11-12)
(1-c) 6M guanidine

The (2-a) and (2-b) indicated below were used as the (2. Allergens and antibodies specific thereto) described above.
(2-a) Purified mite allergens: product name "Der f1" (made by ITEA Inc.) (origin: *Dermatophagoides farinae)*
(2-b) Anti-Der f1 monoclonal antibodies: 5F2.1.8 (made by ITEA Inc.)

A test was conducted using the operational procedure shown in FIG. 5. Specifically, purified mite allergens were dot-blotted in an amount of 0, 12.5, 25, 50, or 100 ng onto a single PVDF membrane cut into a rectangular strip to produce an allergen-supporting membrane. Two identical allergen-supporting membranes were provided in this manner. First, the two allergen-supporting membranes were treated using the same test drug, a washing treatment using PBS was furthermore carried out, and then one of the treated membranes was supplied for protein staining using the gold stain and the other of the treated membranes was supplied for immunostaining using the anti-Der f1 monoclonal antibodies.

FIG. 8 shows results in the form of protein staining images and immunostaining images.

As can be seen in the results of protein staining shown in FIG. 8, with treatment using any of the drugs being examined, the extent of the staining images compares favorably with that in the treatment carried out using PBS (-), and staining images having an intensity corresponding to the dotted quantity of purified mite allergens were obtained. Thus, it was understood that the purified mite allergens did not notably peel away due to the drugs being examined.

On the other hand, as can be seen in the results of immunostaining shown in FIG. 8, when the allergen-supporting membranes were treated using the drugs being examined, the extent of the staining images was lesser overall than that in the treatment carried out using PBS (-), and staining images having an intensity corresponding to the dotted quantity of purified mite allergens were not obtained. Thus, it was understood that the properties of the purified mite allergens supported on the membrane enabling recognition by the specific antibodies (anti-Der f1 monoclonal antibodies) weakened due to the treatments performed using the drugs being examined. Additionally, the extent to which the capacity for recognition by the specific antibodies weakened was different depending on the type of test drug; in particular, the capacity for recognition notably weakened due to 5 ppm of Bi-jia water or 5 ppm of sodium hypochlorite.

<Test example 2>

The (1-a), (1-b), (1-c), or (1-d) indicated below was used as the (1. Test drug) described above.
(1-a) 5 ppm of Bi-jia water (slightly acidic sodium hypochlorite, pH 5.5)
(1-b) 5 ppm of sodium hypochlorite (pH 11-12)
(1-c) 6M guanidine
(1-d) 0.1 w/v% SDS

The (2-a) and (2-b) indicated below were used as the (2. Allergens and antibodies specific thereto) described above.
(2-a) Purified mite allergens: product name "Der f1" (made by ITEA Inc.) (origin: *Dermatophagoides farinae)*
(2-b) Anti-Der f1 monoclonal antibodies: 5F2.1.8 (made by ITEA Inc.)

A test was conducted using the operational procedure shown in FIG. 5. Specifically, purified mite allergens were dot-blotted in an amount of 0, 4, 20, or 100 ng onto a single PVDF membrane cut into a rectangular strip to produce an allergen-supporting membrane. Two identical allergen-supporting membranes were provided in this manner. First, the two allergen-supporting membranes were treated using the same test drug, a washing treatment using PBS was furthermore carried out, and then one of the treated membranes was supplied for protein staining using the gold stain and the other of the treated membranes was supplied for immunostaining using the anti-Der f1 monoclonal antibodies.

FIG. 9 shows results in the form of protein staining images and immunostaining images.

As can be seen in the results of protein staining shown in FIG. 9, with treatment using any of the drugs being examined, the extent of the staining images compares favorably with that in the treatment carried out using PBS (-), and staining images having an intensity corresponding to the dotted quantity of purified mite allergens were obtained. Thus, it was understood that the purified mite allergens did not notably peel away due to the drugs being examined.

On the other hand, as can be seen in the results of immunostaining shown in FIG. 9, when the allergen-supporting membranes were treated using the drugs being examined, the extent of the staining images was lesser overall than that in the treatment carried out using PBS (-), and staining images having an intensity corresponding to the dotted quantity of purified mite allergens were not obtained. Thus, it was understood that the properties of the purified mite allergens supported on the membrane enabling recognition by the specific antibodies (anti-Der f1 monoclonal antibodies) weakened due to the treatments performed using the drugs being examined. Additionally, the extent to which the capacity for recognition by the specific antibodies weakened was different depending on the type of test drug; in particular, the capacity for recognition notably weakened due to 5 ppm of Bi-jia water, 5 ppm of sodium hypochlorite, or 0.1 w/v% SDS.

### <Test example 3>

The (1-e) or (1-f) indicated below was used as the (1. Test drug) described above.
(1-e) 30 v/v% hydrogen peroxide water
(1-f) 10 mM hydrochloric acid (pH 2)

The (2-a) and (2-b) indicated below were used as the (2. Allergens and antibodies specific thereto) described above.
(2-a) Purified mite allergens: product name "Der f1" (made by ITEA Inc.) (origin: *Dermatophagoides farinae)*
(2-b) Anti-Der f1 monoclonal antibodies: 5F2.1.8 (made by ITEA Inc.)

A test was conducted using the operational procedure shown in FIG. 5. Specifically, purified mite allergens were dot-blotted in an amount of 0, 4, 20, or 100 ng onto a single PVDF membrane cut into a rectangular strip to produce an allergen-supporting membrane. Two identical allergen-supporting membranes were provided in this manner. First, the two allergen-supporting membranes were treated using the same test drug, a washing treatment using PBS was furthermore carried out, and then one of the treated membranes was supplied for protein staining using the gold stain and the other of the treated membranes was supplied for immunostaining using the anti-Der f1 monoclonal antibodies.

FIG. 10 shows results in the form of protein staining images and immunostaining images.

As can be seen in the results of protein staining shown in FIG. 10, with treatment using any of the drugs being examined, the extent of the staining images compares favorably with that in the treatment carried out using PBS (-), and staining images having an intensity corresponding to the dotted quantity of purified mite allergens were obtained. Thus, it was understood that the purified mite allergens did not notably peel away due to the drugs being examined.

On the other hand, as can be seen in the results of immunostaining shown in FIG. 10, when the allergen-supporting membranes were treated using the drugs being examined, the extent of the staining images was lesser overall than that in the treatment carried out using PBS (-), and staining images having an intensity corresponding to the dotted quantity of purified mite allergens were not obtained. Specifically, it was understood that the properties of the purified mite allergens supported on the membrane enabling recognition by the specific antibodies (anti-Der f1 monoclonal antibodies) weakened due to the treatments performed using the 30 v/v% hydrogen peroxide water or the 10 mM hydrochloric acid (pH 2).

### <Test example 4>

The (1-a), (1-b), (1-c), (1-d), or (1-e) indicated below was used as the (1. Test drug) described above.
(1-a) 5 ppm of Bi-jia water (slightly acidic sodium hypochlorite, pH 5.5)
(1-b) 5 ppm of sodium hypochlorite (pH 11-12)
(1-c) 6M guanidine
(1-d) 0.1 w/v% SDS
(1-e) 30 v/v% hydrogen peroxide water

The (2-c) and (2-d) indicated below were used as the (2. Allergens and antibodies specific thereto) described above.
(2-c) Purified Japanese cedar pollen allergens: product name "Cry j1" (made by ITEA Inc.) (origin: Japanese cedar (*Cryptomeria japonica*))
(2-d) Anti-Cry j1 monoclonal antibodies: KW-S131 (made by ITEA Inc.)

A test was conducted using the operational procedure shown in FIG. 5. Specifically, purified Japanese cedar pollen allergens were dot-blotted in an amount of 0, 4, 20, or 100 ng onto a single PVDF membrane cut into a rectangular strip to produce an allergen-supporting membrane. Two identical allergen-supporting membranes were provided in this manner. First, the two allergen-supporting membranes were treated using the same test drug, a washing treatment using PBS was furthermore carried out, and then one of the treated membranes was supplied for protein staining using the gold stain and the other of the treated membranes was supplied for immunostaining using the anti-Cry j1 monoclonal antibodies.

FIG. 11 shows results in the form of protein staining images and immunostaining images.

As can be seen in the results of protein staining shown in FIG. 11, with treatment using any of the drugs being examined, the extent of the staining images compares favorably with that in the treatment carried out using PBS (-), and staining images having an intensity corresponding to the dotted quantity of purified Japanese cedar pollen allergens were obtained. Thus, it was understood that the purified Japanese cedar pollen allergens did not notably peel away due to the drugs being examined.

On the other hand, as can be seen in the results of immunostaining shown in FIG. 11, when the allergen-supporting membranes were treated using the drugs being examined, the extent of the staining images was lesser overall than that in the treatment carried out using PBS (-), and staining images having an intensity corresponding to the dotted quantity of purified Japanese cedar pollen allergens were not obtained. Thus, it was understood that the properties of the purified Japanese cedar pollen allergens supported on the membrane enabling recognition by the specific antibodies (anti-Cry j1 monoclonal antibodies) weakened due to the treatments performed using the drugs being examined.

### <Test example 5>

The (1-a), (1-b), or (1-c) indicated below was used as the (1. Test drug) described above.
(1-a) 5 ppm of Bi-jia water (slightly acidic sodium hypochlorite, pH 5.5)
(1-b) 5 ppm of sodium hypochlorite (pH 11-12)
(1-c) 6M guanidine

The (2-a) and (2-b) indicated below were used as the (2. Allergens and antibodies specific thereto) described above.
(2-a) Purified mite allergens: product name "Der f1" (made by ITEA Inc.) (origin: *Dermatophagoides farinae)*
(2-b) Anti-Der f1 monoclonal antibodies: 5F2.1.8 (made by ITEA Inc.)

A test was conducted using the operational procedure shown in FIG. 5. Specifically, purified mite allergens were dot-blotted in an amount of 0, 4, 20, or 100 ng onto a single nitrocellulose membrane cut into a rectangular strip to produce an allergen-supporting membrane. Two identical allergen-supporting membranes were provided in this manner. First, the two allergen-supporting membranes were treated using the same test drug, and then one of the treated membranes was supplied for protein staining using the gold stain and the other of the treated membranes was supplied for immunostaining using the anti-Der f1 monoclonal antibodies.

FIG. 12 shows results in the form of protein staining images and immunostaining images.

As can be seen in the results of protein staining shown in FIG. 12, with treatment using any of the drugs being examined, the extent of the staining images compares favorably with that in the treatment carried out using PBS (-), and staining images having an intensity corresponding to the dotted quantity of purified mite allergens were obtained. Thus, it was understood that the purified mite allergens did not notably peel away due to the drugs being examined.

In the present test example, in which a nitrocellulose membrane was used, a signal corresponding to the quantity of allergens used was comparatively weaker than in the results of test examples 1 to 4, in which a PVDF membrane was used, which suggested that the capability of the nitrocellulose membrane to support purified mite allergens is weaker than that of the PVDF membrane.

On the other hand, as can be seen in the results of immunostaining shown in FIG. 12, when the allergen-supporting membranes were treated using the drugs being examined, the extent of the staining images was lesser overall than that in the treatment carried out using PBS (-), and staining images having an intensity corresponding to the dotted quantity of purified mite allergens were not obtained. Thus, it was understood that the properties of the purified mite allergens supported on the membrane enabling recognition by the specific antibodies (anti-Der f1 monoclonal antibodies) weakened due to the treatments performed using the drugs being examined.

### <Test example 6>

The (1-a), (1-b), or (1-c) indicated below was used as the (1. Test drug) described above.
(1-a) 5 ppm of Bi-jia water (slightly acidic sodium hypochlorite, pH 5.5)
(1-b) 5 ppm of sodium hypochlorite (pH 11-12)
(1-c) 6M guanidine

The (2-a) and (2-b) indicated below were used as the (2. Allergens and antibodies specific thereto) described above.
(2-a) Purified mite allergens: product name "Der f1" (made by ITEA Inc.) (origin: *Dermatophagoides farinae)*
(2-b) Anti-Der f1 monoclonal antibodies: 5F2.1.8 (made by ITEA Inc.)

A test was conducted in the same manner as with the operational procedure shown in FIG. 5, except that three allergen-supporting membranes were prepared. Specifically, purified mite allergens were dot-blotted in an amount of 0, 25, 50, or 100 ng onto a single PVDF membrane cut into a rectangular strip to produce an allergen-supporting membrane. Three identical allergen-supporting membranes were provided in this manner. First, the three allergen-supporting membranes were treated using the same test drug, a washing treatment using PBS was furthermore carried out, and then one of the treated membranes was supplied for protein staining using the gold stain, another of the treated membranes was supplied for protein staining using the high-sensitivity CBB stain, and yet another of the treated membranes was supplied for immunostaining using the anti-Der f1 monoclonal antibodies.

FIG. 13 shows results in the form of protein staining images and immunostaining images.

As can be seen in the results of protein staining using the gold stain and the high-sensitivity CBB stain shown in FIG. 13, with treatment using any of the drugs being examined, the extent of the staining images between each of the drugs being examined compares favorably with that in the treatment carried out using PBS (-), and staining images having an intensity corresponding to the dotted quantity of purified mite allergens were obtained.

When the high-sensitivity CBB stain was used in the protein staining, a visual signal corresponding to the quantity of allergens used was comparatively weaker than in the results in which the gold stain was used in the protein staining, which suggested that the capability of the high-sensitivity CBB stain to detect purified mite allergens is weaker than that of the gold stain.

On the other hand, as can be seen in the results of immunostaining shown in FIG. 13, when the allergen-supporting membranes were treated using the drugs being examined, the extent of the staining images was lesser overall than that in the treatment carried out using PBS (-), and staining images having an intensity corresponding to the dotted quantity of purified mite allergens were not obtained. Thus, it was understood that the properties of the purified mite allergens supported on the membrane enabling recognition by the specific antibodies (anti-Der f1 monoclonal antibodies) weakened due to the treatments performed using the drugs being examined.

### <Test example 7>

The (1-d) or (1-g) indicated below was used as the (1. Test drug) described above.
(1-d) 0.1 w/v% SDS
(1-g) 70 v/v% 2-propanol-containing water

The (2-a) and (2-b) indicated below were used as the (2. Allergens and antibodies specific thereto) described above.
(2-a) Purified mite allergens: product name "Der f1" (made by ITEA Inc.) (origin: *Dermatophagoides farinae)*
(2-b) Anti-Der f1 monoclonal antibodies: 5F2.1.8 (made by ITEA Inc.)

### •On-membrane reaction

The allergens were treated using the test drug on the allergen-supporting membrane in the same manner as in test examples 1 to 6. Specifically, testing was conducted as described below.

A test was conducted using the operational procedure shown in FIG. 5. Specifically, purified mite allergens were dot-blotted in an amount of 0, 4, 20, or 100 ng onto a single PVDF membrane cut into a rectangular strip to produce an allergen-supporting membrane. Two identical allergen-supporting membranes were provided in this manner. First, the two allergen-supporting membranes were treated using the same test drug, a washing treatment using PBS was furthermore carried out, and then one of the treated membranes was supplied for protein staining using the gold stain and the other of the treated membranes was supplied for immunostaining using the anti-Der f1 monoclonal antibodies.

### •In-solution reaction

The allergens were treated using the test drug in a solution obtained by mixing the allergens and the test drug. Specifically, testing was conducted as described below.
- A substance in which the concentration of an undiluted solution of allergens was adjusted using the test drug (50 ng/uL), a substance obtained by diluting the resultant substance to 1/5 concentration using the test drug (10 ng/µL), and a substance obtained by diluting the resultant substance to 1/5 concentration using the test drug (2 ng/µL) were each provided and allowed to stand for two hours at normal temperature.
- A PVDF membrane was immersed in methanol to be made hydrophilic, after which 2 µL of either the aforementioned reaction solutions or the test drug was blotted in a dot formation (0-100 ng/dot).
- Protein staining using a gold stain or immunostaining using anti-Der f1 monoclonal antibodies was carried out.

FIG. 14 shows results in the form of protein staining images and immunostaining images for each of the on-membrane reactions and in-solution reactions.

As can be seen in the results of protein staining for the on-membrane reaction shown in FIG. 14, with treatment using any of the drugs being examined, the extent of the staining images between each of the drugs being examined compares favorably with that in the treatment carried out using PBS (-), and staining images having an intensity corresponding to the dotted quantity of purified mite allergens were obtained.

By contrast, as can be seen in the results of protein staining for the in-solution reaction shown in FIG. 14, when the allergens were treated using the test drug in a solution obtained by mixing the allergens and the test drug, the extent of the staining images was lesser overall than that in the treatment carried out using PBS (-), and staining images having an intensity corresponding to the dotted quantity of purified mite allergens were not obtained. Thus, it was understood that even if the purified mite allergens were tried to support on a PVDF membrane after having been treated using the test drug, the purified mite allergens were affected by the test drug and could not be adequately supported.

On the other hand, as can be seen in the results of immunostaining for the on-membrane reaction shown in FIG. 14, when the allergen-supporting membranes were treated using the drugs being examined, the extent of the staining images was lesser overall than that in the treatment carried out using PBS (-), and staining images having an intensity corresponding to the dotted quantity of purified mite allergens were not obtained. Thus, it was understood that the properties of the purified mite allergens supported on the membrane enabling recognition by the specific antibodies (anti-Der f1 monoclonal antibodies) weakened due to the treatments performed using the drugs being examined.

By contrast, as can be seen in the results of immunostaining for the in-solution reaction shown in FIG. 14, because the purified mite allergens could not be adequately supported on the PVDF membrane due to the treatment using the test drug in the in-solution reaction, it was also impossible to obtain an immunostaining signal. Specifically, it became clear that, using the method for in-solution reactions, it was impossible to distinguish and evaluate whether the test drug affects properties for supporting the allergens on the membrane or affects antibody recognition properties.

### <Test example 8>

A test for evaluating allergen inactivators was conducted using, as an allergen support, a multiscreen filter plate (MultiScreen HTS HA sterile plate, Millipore, #MSHAS4510) provided with a filter membrane composed of a cellulose mixed ester (substance similar to nitrocellulose) in each well. Specifically, as per the main points of the operational procedure for the test shown in FIG. 6, a test was conducted in accordance with the following protocol.

(1. Formulation of allergen-supporting membrane and treatment carried out using test drug)
   - An amount of 2 µL of purified Japanese cedar pollen allergens (product name "Cry j1") (made by ITEA Inc.) (origin: Japanese cedar (*Cryptomeria japonica*)) in each stage of dilution (50, 10, 2, or 0 ng/uL) was added dropwise into each well of the multiscreen filter plate and was allowed to spontaneously dry.
   - Filtration washing was carried out three times using 200 µL of PBS.
   - An amount of 200 µL of the drugs being examined (the various drugs being examined, and concentrations thereof, shown in FIG. 15; PBS as a control) was added, and the multiscreen filter plate was allowed to stand for two hours at normal temperature.
   - The drugs being examined were removed through pipetting.
   - Filtration washing was carried out three times using 200 µL of PBS.
   - To immunostaining or gold staining
(2. Immunostaining)
   - An amount of 200 µL of 1 w/v% gelatin-containing PBS was added as a blocking buffer, and blocking was carried out for 15 minutes at normal temperature.
   - The blocking buffer was removed through filtration.
   - Filtration washing was carried out three times using 200 µL of PBS.
   - Anti-Cry j1 monoclonal antibodies (S10) (made by ITEA Inc.) serving as primary antibodies were diluted using 1 w/v% BSA-containing PBST (0.02 v/v% Tween-20) so as to reach a final concentration of 0.5 µg/mL, 200 µL of the primary antibodies was added, and a reaction was carried out for 30 minutes at normal temperature.
   - The primary antibodies were removed through filtration.
   - Filtration washing was carried out three times using 200 µL of PBS.
   - HRP-labeled secondary antibodies (product name "HRP-Goat anti-Mouse IgG," made by Zymed Laboratories) serving as secondary antibodies were diluted using 1 w/v% BSA-containing PBST (0.02 v/v% Tween-20) so as to reach a concentration of 0.75 µg/mL, 200 µL of the secondary antibodies was added, and a reaction was carried out for 30 minutes at normal temperature.
   - The secondary antibodies were removed through filtration.
   - Filtration washing was carried out four times using 200 µL of PBS.
   - An amount of 35 µL of a coloring reagent solution (product name "EZ West Blue," made by ATTO Co., Ltd.) was added, and a reaction was carried out for ten minutes at normal temperature.
   - The coloring reagent solution was removed through filtration.
   - Filtration washing was carried out three times using 200 µL of distilled water, after which the membrane was allowed to spontaneously dry.
(3. Gold staining)
   A gold stain kit (product name "Gold protein detection kit for membranes," made by Cytodiagnostics Inc.) was used.
   - An amount of 200 µL of a "1X wash buffer" included in the kit was added, and a reaction was carried out for 15 minutes at normal temperature.
   - The "1X wash buffer" was removed through filtration.
   - Filtration washing was carried out twice using 200 µL of distilled water.
   - An amount of 200 µL of a "Gold Solution" included in the kit was added, and a reaction was carried out for one hour at normal temperature.
   - The "Gold Solution" was removed through pipetting.
   - Washing (pipetting) was carried out three times using 200 µL of the "1X wash buffer).
   - Filtration washing was carried out three times using 200 µL of distilled water, after which the membrane was allowed to spontaneously dry.

FIG. 15 shows results in the form of gold staining images and immunostaining images.

As can be seen in the results of the gold staining shown in FIG. 15, with treatment using any of the drugs being examined, the extent of the staining images compares favorably with that in the treatment carried out using PBS (-), and staining images having an intensity corresponding to the dotted quantity of purified Japanese cedar pollen allergens were obtained. Thus, it was understood that the purified Japanese cedar pollen allergens did not notably peel away due to the drugs being examined.

On the other hand, as can be seen in the results of immunostaining shown in FIG. 15, when the allergen-supporting membranes were treated using the drugs being examined, the extent of the staining images was lesser overall than that in the treatment carried out using PBS (-), and staining images having an intensity corresponding to the dotted quantity of purified Japanese cedar pollen allergens were not obtained. Thus, it was understood that the properties of the purified Japanese cedar pollen allergens supported on the membranes provided to the multiscreen filter enabling recognition by the specific antibodies (anti-Cry j1 monoclonal antibodies) weakened due to the treatments performed using the drugs being examined. Additionally, the extent to which the capacity for recognition by the specific antibodies weakened was different depending on the type of test drug; in particular, the capacity for recognition notably weakened due to 5 ppm of NaClO (Bi-jia water) or 6M guanidine.

### <Test example 9>

A test for evaluating allergen inactivators was conducted using, as an allergen support, a multiscreen filter plate (MultiScreen HTS IP, 0.45 µm, Millipore, #MSIPS4510) provided with a filter membrane composed of PVDF in each well. Specifically, as per the main points of the operational procedure for the test shown in FIG. 6, a test was conducted in accordance with the following protocol.

(1. Formulation of allergen-supporting membrane and treatment carried out using test drug)
   - An amount of 10 µL of 35 v/v% ethanol water was added to each well in the multiscreen filter plate to make the multiscreen filter plate hydrophilic, and then filtration washing was immediately carried out twice using 200 µL of PBS.
   - An amount of 2 µL of purified Japanese cedar pollen allergens (product name "Cry j1") (made by ITEA Inc.) (origin: Japanese cedar (*Cryptomeria japonica*)) in each stage of dilution (50, 10, 2, or 0 ng/µL) was added dropwise and was suctioned through filtration.
   - Filtration washing was carried out three times using 200 µL of PBS.
   - An amount of 200 µL of the drugs being examined (the various drugs being examined, and concentrations thereof, shown in FIG. 16; PBS as a control) was added, and the multiscreen filter plate was allowed to stand for two hours at normal temperature.
   - The drugs being examined were removed through pipetting.
   - Filtration washing was carried out three times using 200 µL of PBS.
   - To immunostaining or gold staining
(2. Immunostaining)
   The same procedure as in test example 8 was carried out.
(3. Gold staining)
   The same procedure as in test example 8 was carried out.

FIG. 16 shows results in the form of gold staining images and immunostaining images.

As can be seen in the results of the gold staining shown in FIG. 16, with treatment using any of the drugs being examined, the extent of the staining images compares favorably with that in the treatment carried out using PBS (-), and staining images having an intensity corresponding to the dotted quantity of purified Japanese cedar pollen allergens were obtained. Thus, it was understood that the purified Japanese cedar pollen allergens did not notably peel away due to the drugs being examined.

On the other hand, as can be seen in the results of immunostaining shown in FIG. 16, when the allergen-supporting membranes were treated using the drugs being examined, the extent of the staining images was lesser overall than that in the treatment carried out using PBS (-), and staining images having an intensity corresponding to the dotted quantity of purified Japanese cedar pollen allergens were not obtained. Thus, it was understood that the properties of the purified Japanese cedar pollen allergens supported on the PVDF membranes provided to the multiscreen filter enabling recognition by the specific antibodies (anti-Cry j1 monoclonal antibodies) weakened due to the treatments performed using the drugs being examined. Additionally, the extent to which the capacity for recognition by the specific antibodies weakened was different depending on the type of test drug; in particular, the capacity for recognition notably weakened due to 0.1% SDS or 6M guanidine.

### <Test example 10>

A test for evaluating allergen inactivators was conducted in the same manner as those conducted in test examples 1 to 7, except that charged-microparticle water dispersed in a gas within a prescribed space was used as a substance being examined. Specifically, as per the main points of the operational procedure for the test shown in FIG. 7, purified mite allergens or purified Japanese cedar pollen allergens were dot-blotted in an amount of 0, 4, 20, or 100 ng onto a single PVDF membrane cut into a rectangular strip to produce an allergen-supporting membrane. Two identical allergen-supporting membranes were provided in this manner. First, the two allergen-supporting membranes were allowed to stand for two hours in a hermetically sealed container in which charged-microparticle water was generated using a device 25, a washing treatment using PBS was furthermore carried out, and then one of the treated membranes was supplied for protein staining using the gold stain and the other of the treated membranes was supplied for immunostaining using the anti-Der f1 monoclonal antibodies or the anti-Cry j1 monoclonal antibodies.

The charged-microparticle water disposed in air within a prescribed space that is used as a substance being examined in the present test example was generated through the method disclosed in the description in the pamphlet for International Publication No. 2004105958.

FIG. 17 shows results in the form of gold staining images and immunostaining images.

As can be seen in the results of the gold staining shown in FIG. 17, even with treatment using the charged-microparticle water dispersed in a gas within a prescribed serving as a substance being examined, the extent of the staining images compares favorably with that when treatment was not carried out, and staining images having an intensity corresponding to the dotted quantity of purified mite allergens or purified Japanese cedar pollen allergens were obtained. Thus, it was understood that the purified mite allergens or purified Japanese cedar pollen allergens did not notably peel away due to the treatments performed using the charged-microparticle water.

On the other hand, as can be seen in the results of immunostaining shown in FIG. 17, when the allergen-supporting membranes were treated using the charged-microparticle water as the substance being examined, the extent of the staining images was lesser overall than that when treatment was not carried out, and staining images having an intensity corresponding to the dotted quantity of purified mite allergens or purified Japanese cedar pollen allergens were not obtained. Thus, it was understood that the properties of the purified mite allergens or purified Japanese cedar pollen allergens supported on the PVDF membranes enabling recognition by the specific antibodies (anti-Cry j1 monoclonal antibodies) weakened due to the treatments performed using the charged-microparticle water.

Thus, it became clear that the evaluation method according to the present invention can be applied even to substances dispersed in a gas that are generated by an air-cleaner device, etc.

### KEY

- 1, 1a: Membrane
- 2: Allergens supported in dot formation
- 3: Multiscreen filter plate
- 3a: End section
- 4: Well
- 4a: Well wall
- 5: Well hole
- 20: Vacuum manifold
- 20a: Flange part
- 21: Space
- 22: Waste liquid recovery container
- 23: Suction port tube
- 24: Connector
- 25: Device for generating charged-microparticle water

## Claims

1. A method for evaluating allergen inactivators, said method being **characterized by** comprising:
a step for preparing an allergen-supporting membrane configured by supporting allergens on a membrane for supporting allergens;
a step for treating the allergen-supporting membrane using a test drug;
a step for washing the allergen-supporting membrane treated using the test drug, and subsequently treating the allergen-supporting membrane using antibodies specific to the allergens;
a step for washing the allergen-supporting membrane treated using the antibodies specific to the allergens, and subsequently detecting the specific antibodies bound to the allergens; and
a step for assessing the extent to which the allergens are inactivated by the test drug based on the detected quantity of the specific antibodies.

2. A method for evaluating allergen inactivators, said method being **characterized by** comprising:
a step for preparing, as a first allergen-supporting membrane, a membrane configured by supporting allergens on a first membrane for supporting allergens;
a step for preparing a second allergen-supporting membrane configured by supporting allergens on a second membrane for supporting allergens in a quantity corresponding to the quantity of allergens supported on the first allergen-supporting membrane;
a step for treating the first allergen-supporting membrane using a test drug;
a step for washing the first allergen-supporting membrane treated using the test drug, and subsequently treating the first allergen-supporting membrane using antibodies specific to the allergens;
a step for washing the first allergen-supporting membrane treated using the antibodies specific to the allergens, and subsequently detecting the specific antibodies bound to the allergens;
a step for treating the second allergen-supporting membrane using a protein staining agent;
a step for washing the second allergen-supporting membrane treated using the protein staining agent, and subsequently detecting the amount of staining by the protein staining agent, the amount corresponding to the quantity of allergens supported on the second allergen-supporting membrane; and
a step for assessing the extent to which the allergens are inactivated by the test drug based on the relationship between the amount of staining and the detected quantity of the specific antibodies.

3. The evaluation method according to claim 2, wherein:
the method furthermore comprises a step for treating the second allergen-supporting membrane using the test drug; and
the second allergen-supporting membrane, which is supplied to the step for treatment using the protein staining agent, is supplied to the step for treatment using the protein staining agent after having been subjected to the step for treatment using the test drug.

4. The evaluation method according to claim 2 or 3, wherein the protein staining agent is a gold staining agent or a high-sensitivity CBB staining agent.

5. The evaluation method according to any of claims 1 to 4, wherein the membrane for supporting allergens is a PVDF-based membrane or a nitrocellulose-based membrane.

6. The evaluation method according to any of claims 1 to 5, wherein the allergens are supported, across a plurality of locations with a varied quantity of allergens, on a single membrane for supporting allergens.

7. The evaluation method according to any of claims 1 to 6, wherein a plurality of types of allergens are supported, across a plurality of locations, on a single membrane for supporting allergens.

8. The evaluation method according to any of claims 1 to 7, wherein the membrane for supporting allergens is formed in sections across a plurality of locations on a plate, and is caused to support the allergens across a plurality of locations formed in sections on the plate with a varied quantity of allergens.

9. The evaluation method according to any of claims 1 to 8, wherein the membrane for supporting allergens is formed in sections across a plurality of locations on a plate, and is caused to support a plurality of types of allergens across a plurality of locations formed in sections on the plate.

10. A kit for evaluating allergen inactivators, said kit comprising a membrane for supporting allergens, specific antibodies for detecting allergens, and a protein staining agent.

11. The evaluation kit according to claim 10, wherein the protein staining agent is a gold staining agent or a high-sensitivity CBB staining agent.

12. The evaluation kit according to claim 10 or 11, wherein the membrane for supporting allergens is a PVDF-based membrane or a nitrocellulose-based membrane.

13. The evaluation kit according to any of claims 10 to 12, wherein the membrane for supporting allergens is formed in the bottom surface of each of a plurality of wells in sections on a plate.

14. The evaluation kit according to any of claims 10 to 13, wherein allergens are supported in advance on the membrane for supporting allergens.
